# EUROPEAN PATENT SPECIFICATION

(11) **EP 1 629 806 B1**
(45) Date of publication and mention of the grant of the patent: **03.12.2008**
(21) Application number: 04020176.6
(22) Date of filing: 25.08.2004
(51) Int. Cl.: A61F 11/08

(54) **Hearing protection earplug and method for manufacturing such an earplug**
Gehörschutzstöpsel und Verfahren zu dessen Herstellung
Bouchon d'oreille et son procédé de fabrication

(43) Date of publication of application: 01.03.2006
(73) Proprietor: PHONAK AG, 8712 Stäfa (CH)
(72) Inventor: Haussmann, Mathias, 8005 Zürich (CH); Meier, Hilmar, 8008 Zürich (CH)
(74) Representative: Schwan - Schwan - Schorer

(56) References cited:
- EP-A- 0 336 487
- WO-A-02/24127
- WO-A-96/40479
- DE-A- 10 041 733
- DE-A- 10 158 648
- GB-A- 925 889
- US-A- 5 488 961

## Description

The invention relates to a hearing protection earplug and to a method for manufacturing such an earplug.

A large part of the population is exposed to hazardous noise from time to time. This can be at work, whilst traveling, during leisure activities or at home. The exposure can lead to permanent hearing loss, distract people's attention from other hazards or simply cause stress. In order to prevent both accidents and permanent hearing damage, hearing protection devices (HPDs) have been provided in many styles and over many years. It started with the earmuff which is still very relevant and addresses very noisy environments (e.g. airports, construction, shooting) or complex working/communication situations (e.g. fighter pilots). Over the years development of biocompatible soft materials has enabled soft earplugs in different styles and colors as well as recent development of "one fits many" standard semi-soft earplugs in silicon-rubber type materials. For severe situations even the combination of an earmuff and an "in-the-ear" HPD is required to achieve desired attenuation. The physical limitation of hearing protection based on ear worn devices is defined where bone-conduction (body acoustics) becomes dominant at around 40dB attenuation.

A common disadvantage of the above mentioned HPD styles is wearing discomfort. In case of the earmuffs, they are large which creates difficulties in combination with other head worn gear and they "close off" the ear too much for most applications. The in-the-ear styles mentioned are devices made to fit "the average" ear in one way or the other. Either the fit is provided by softness of the material which leads to undefined device insertion and undefined attenuation, or the fit is provided by standard shaped structures intended to block off the ear canal. In both cases the flat distribution of the individual shape of the outer ear and the ear canal leads to bad fit, pressure points in the ear and undefined positioning of the device.

To address this wearing comfort issue, in-the-ear hearing aid technology has been applied making customized ear molds with passive acoustical filter. These are long lasting devices with good wearing comfort. However, this customization process is traditionally a very manual process creating varying results over time, low reproducibility and the quality is very operator skill dependent.

DE 100 41 733 A1 relates to a hearing protection earplug according the preamble of claim 1, consisting of a hollow shell having a wall thickness of about 1 mm and extending close to the ear drum of the user, wherein a thin barrier with a filter insert is provided close to the ear drum for attenuating sound transmission through the shell. The large cavity between the outer opening of the shell and the filter insert is provided for at least partly maintaining the natural resonance of the ear canal which has an individual resonance frequency between 2.5 kHz and 3 kHz. The outer surface of the shell is individually shaped according to the measured user's ear anatomy.

DE 299 05 634 U1 relates a similar hearing protection earplug, wherein a hollow shell having an outer surface individually shaped according to the measured user's ear anatomy is provided with a filter tube at the distal end of the inner cavity of the hollow shell.

EP 0 336 487 B1 relates to a hearing protection earplug comprising a maze-like acoustic filter having a plurality of series-connected resonance cavities for providing for an effective sound attenuation. The filter is located at the outer opening of a shell to be worn in the person's ear canal.

EP 0 315 942 A1 relates to a hearing protection earplug comprising as diaphragm filter for sound attenuation between an outer opening of a shell and an inner tube which is connected to the shell. The diaphragm and the tube are designed to restore the natural resonance of the ear canal.

US 6,148,821 relates to a hearing protection earplug which is tuneable by the user and comprises an acoustic filter within an inner channel connecting an outer opening of the earplug with an inner opening at the distal end of the earplug.

US 5,832,094 relates to a hearing protection earplug which has an outer surface individually shaped according to the user's ear anatomy and which is provided with one or two Helmholtz resonance cavities at the outer opening of the earplug which act as a filter for effective sound attenuation.

US 5,333,622 relates to a hearing protection earplug having an outer surface individually shaped according to the user's ear anatomy which comprises an embedded Helmholtz resonator cavity.

CH 287384 relates to a hearing protection earplug which comprises an outer sound inlet opening and a cavity between the opening and two channels which may be arranged in parallel or in series and which are designed as a sound attenuation filter which has a relatively low attenuation for frequencies which are relevant for speech perception.

US 5,113,967 relates to two-part hearing protection earplug consisting of a distal portion to be inserted into the user's outer ear canal and a proximal cap-like portion with a sound input opening at its distal end, a resonance channel having a first portion extending from the sound input opening to the proximal end of the cap-like portion and a second portion coaxial to the first portion and extending from this proximal end to a passive acoustic filter located at the distal end of the cap-like portion. The resonance channel has a resonance around 2.7 kHz and serves to reduce the sound attenuation by the earplug at high frequencies between 2 kHz and 10 kHz.

GB 925,889 and EP 0 336 487 A1 relate to earplugs comprising a sound attenuation filter having a maze-like structure.

It is an object of the invention to provide for a hearing protection earplug which provides for an optimized hearing protection function for the individual person using the earplug, wherein both a safe hearing protection function and nevertheless a close-to-natural hearing impression should be achieved.

The above object is achieved by an earplug as defined in claim 1 and a corresponding manufacturing method as defined in claim 19.

The invention is beneficial in that, by providing the hearing protection earplug with a resonance cavity which is individually designed according to a desired sound frequency response of the person's outer ear and ear canal when wearing the earplug, the sound frequency response provided by the earplug can be optimized for the individual user of the earplug. In particular, by individually shaping the resonance cavity, it is possible to obtain a sufficient sound attenuation while at the same time undesired excess attenuation in particular frequency ranges, namely in the frequency range of speech, can be avoided so that speech communication is enabled at least to some extent despite the sound attenuation function provided by the earplug. The inner mechanical structure of the resonance cavity is designed such that the effective acoustic length between the outer opening of the shell and the filter element is larger than the geometrical distance between the outer opening and the filter element.

The resonance cavity is designed such that the specific natural sound frequency response of the user's outer ear and ear canal is restored at least to some extent, so that the sound frequency response of the person's outer ear and ear canal, when wearing the earplug, is substantially equal to the corresponding natural sound frequency response.

Preferred embodiments are defined in the dependent claims.

In the following, examples of the invention will be illustrated by reference to the attached drawings.
Fig. 1 shows a schematic longitudinal sectional view of an example of an earplug according to the invention when inserted into a person's ear canal;
Fig. 2 shows a view like Fig. 1 of a modified embodiment; and
Fig. 3 shows a schematic longitudinal sectional view of another example of an earplug according to the invention.

In general, it would be desirable to have a hearing protection device which provides for sufficient attenuation of undesired sound signals, such as noise, while preserving a more or less natural hearing impression for desired sound signals, such as natural speech, in order to enable speech communication in noisy environments. In other words, it would be desirable to have more or less frequency independent attenuation in order to achieve a close to natural hearing impression. In this respect in is important to note that the outer ear and the ear canal of a person have an individual natural resonance around 2.5 kHz to 3 kHz so that natural speech for which significant information is carried in this frequency region is selected over other sound signals such as noise. Technically, this natural resonance is due to the fact that the length of the ear canal equals about one quarter of the wave length of a standing wave corresponding to the resonance frequency. Thus, a wavelength of about four times the length of the ear canal corresponds to a resonance frequency of about 2.5 kHz.

This natural frequency of the ear canal will be disturbed by any earplug worn in the ear canal. Even if the earplug primarily consists of a hollow shell such as described in DE 299 05 635 U1 or DE 100 41 733 A1, which is substantially closed at its distal end by a sound attenuation filter, the effective acoustical length of the ear canal will be shortened, for example, cut in approximately two equal parts. Thereby the resonance frequency would be increased, for example, by a factor of two if the ear canal is cut in two equal parts. Thus, even when using a hollow shell as the earplug, the natural hearing impression would be disturbed and speech signals would be more difficult to be heard and understood by the user of the earplug.

The embodiments shown in the figures address this problem by compensating the shortening of the ear canal by the earplug by correspondingly increasing the effective acoustic length within the earplug. In other words, if the cavity of the earplug is dimensioned such that the ear canal is effectively cut in two equal parts, the cavity of the earplug would be designed such that the effective acoustic length within the cavity is about double the geometrical length of the cavity.

In this respect, the term "effective acoustic length" is intended to designate the actual travel distance of a sound wave between two points A and B, while the term "geometrical length" or "geometrical distance" is intended to designate the shortest or direct distance between the two points A and B.

The basic idea is to provide a resonance cavity between an outer opening of the earplug and a sound attenuation filter at the distal end of the earplug, which is provided with an inner mechanical structure which serves to tune the resonance frequency of the resonance cavity. Thereby it becomes possible to individually design the resonance cavity and hence the earplug in such a manner that the sound frequency response of the user's outer ear and ear canal, when wearing the earplug, may be adapted to a desired sound frequency response. In other words, thereby the acoustic response of the earplug can be designed in a simple manner in order to achieve a desired sound frequency response for the individual user.

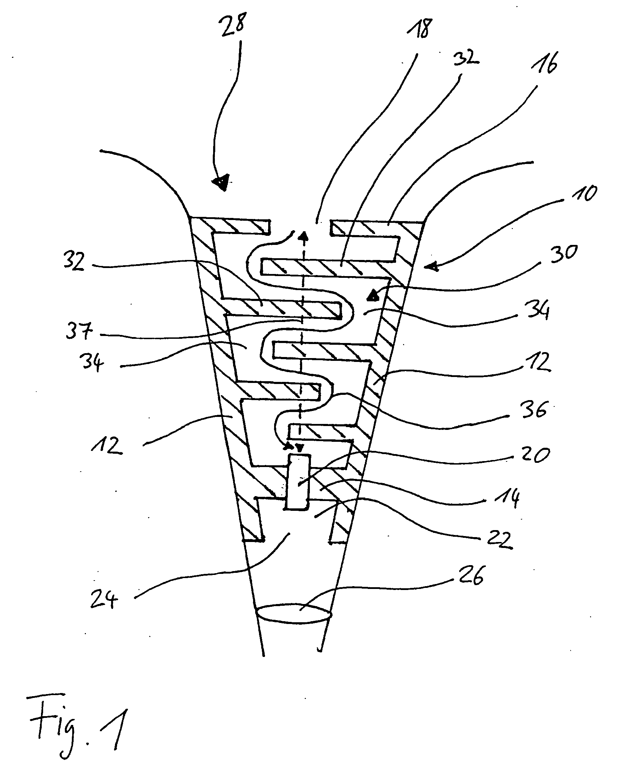

Fig. 1 shows an earplug comprising a relatively hard shell 10 which preferably may have an elasticity of from shore D85 to shore D65 and may be made, for example, of polyamide. The shell 10 comprises a lateral wall 12, a distal wall 14 and a proximal wall (or faceplate) 16. The proximal wall 16, i.e. the outer end of the shell 10, is provided with an outer opening 18 for sound input. The distal wall 14 is provided with a sound attenuation filter 20 which penetrates through the wall 14. At the distal side of the distal wall 14 a distal cavity 22 having an inner opening 24 is formed which faces the ear drum 26 of the user, when the shell 10 is inserted into the ear canal 28 of the person using the earplug. A resonance cavity 30 is formed between the outer opening 18 and the attenuation filter 20, which is limited by the walls 12, 14 and 16. The resonance cavity 30 does not extend beyond the proximal wall 16 and is provided with an inner mechanical structure enabling to individually tune the resonance frequency of the resonance cavity 30.

In the embodiment shown in Fig. 1 the inner mechanical structure of the resonance cavity 30 is a maze-like structure consisting of a plurality of axially spaced apart lamella-like elements 32 extending from the lateral wall 12 radially into the resonance cavity 30, wherein each element 32 has at least one sound opening 34. The sound openings 34 of adjacent elements 32 are radially spaced apart from each other. In the embodiment shown in Fig. 1 all sound openings 34 may have about the same size and shape so that the resonance cavity 30 is similar, to some extent, to a tube-like structure. Preferably, the size and shape of the sound openings 34 are at least similar to that of the outer opening 18.

Due to this maze-like structure, the effective acoustic length of the cavity 30, i.e. the length of the travel path 36 the sound waves have to take through the resonance cavity 30, is significantly larger than the geometrical distance 37 between the outer opening 18 and the filter 20 (see dashed line in Fig. 1). Preferably, this increase is at last 50%. The effective acoustic length 36 may be selected such that it corresponds to about one quarter of the wavelength of a standing wave corresponding to the desired basic resonance frequency of the resonance cavity 30.

In general the resonance frequency obtained in this manner should be between 800 Hz and 5 kHz.

By providing for such a specific resonance cavity 30, a sound frequency response of the user's outer ear and ear canal, when wearing the earplug, may be achieved which has a maximum between 800 Hz and 5 kHz. Thereby a frequency response may be obtained which is substantially equal to the corresponding natural sound frequency response of the user's outer ear and ear canal when not wearing the earplug.

In general, the inner structure of the resonance cavity 30 may serve to "emulate" the original or non-occluded length of the person's ear canal 28 in order to reconstruct the natural acoustic resonance of the non-occluded ear. However, for achieving specific other functionality, an effective acoustic length of the resonance cavity 30 may be selected which is different from the non-occluded length of the person's ear canal 28.

The filter 20 is designed for acoustically connecting the resonance cavity 30 and the distal cavity 22 and maybe, for example, a tube filter or a diaphragm filter.

Before manufacturing the shell 10, the inner shape of the user's outer ear and ear canal is measured, for example by three-dimensional laser scanning or by forming an impression of the ear which subsequently undergoes three-dimensional laser scanning. Preferably, the outer surface of the shell 10 is individually shaped according to the measured inner shape of the user's outer ear and ear canal in order to optimize fitting of the shell 10 within the ear canal 28. Preferably, the shell 10 is built up by an additive process, such as layer-by-layer laser sintering of a powder material. Examples for such processes are given, for example, in US 2003/0133583 A1 or in US 6,533,062 B1.

Further, before manufacturing the shell 10, a desired sound frequency response of the user's outer ear and ear canal when wearing the earplug is individually determined. Optionally, also a hearing loss of the user may be determined.

As already mentioned above, the measured shape of the user's out ear and ear canal on the one hand is used for determining the desired outer shape of the shell 10 for optimizing fitting within the ear canal 28. On the other hand, however, the above-mentioned individual data of the user is input into an acoustic model which serves to determine a set of geometrical parameters of the shell 10 in order to tune the sound frequency response of the earplug according to the desired sound frequency response of the user's outer ear and ear canal when wearing the earplug. Part of these parameters is used for defining the outer shape of the shell 10. In addition, however, part of the parameters is also used for determining the inner shape of the resonance cavity 30. In this respect, important parameters are the diameter of the outer opening 18, the volume of the resonance cavity 30 and the effective acoustic length 36 between the outer opening 18 and the filter 20. By varying these parameters, the resonance frequency and also the quality factor of the resonance cavity 30 may be tuned individually according to the desired frequency response.

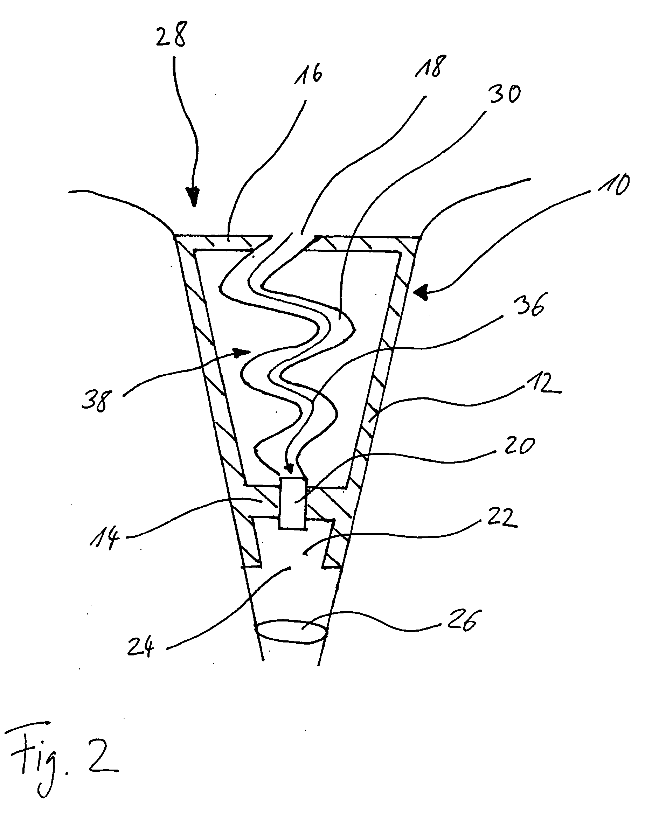

Fig. 2 shows a modification of the embodiment of Fig. 1 wherein the inner structure of the resonance cavity 30 which determines the effective acoustic length 36 is formed by a curved tube 38 which extends through the shell 10 from the outer opening 18 to the attenuation filter element 20. In this embodiment, the resonance cavity 30 is formed by the interior of the tube 38, with the effective acoustic length 36 corresponding to the length of the tube 38.

Although the embodiments of Figs. 1 and 2 relate to passive hearing protection earplugs, the invention in general also may be applied to the passive acoustic attenuation filter aspect of active hearing protection earplugs, i.e. earplugs including a microphone, an audio signal processing unit, such as an amplifier, and a speaker for frequency-selective or temporary bypassing of the acoustic attenuation filter function provided by the earplug, for example, in order to enable acoustic communication with other persons in noisy environments.

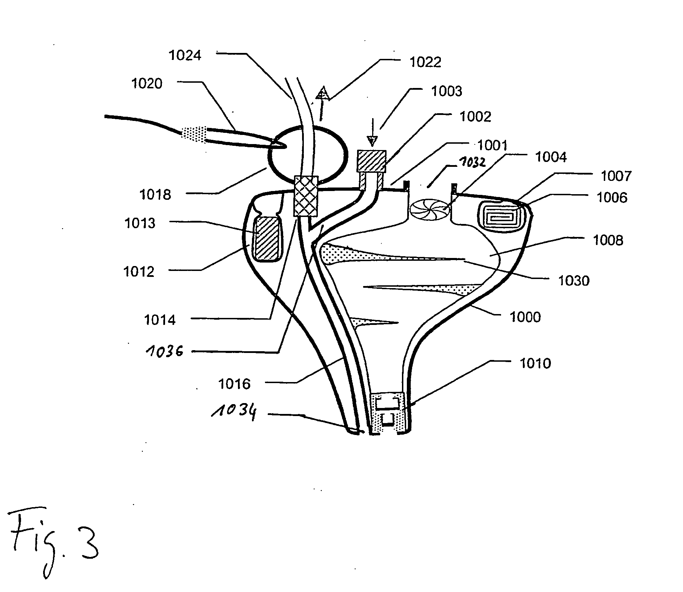

Fig. 3 shows an example of a passive hearing protection earplug according to the invention wherein a resonance cavity 1008 with an inner mechanical structure 1030 for frequency tuning designed similar to that of Fig. 1 is combined with other features which may be advantageously implemented by manufacturing the shell of the earplug by an additive buildup process, such as layer-by-layer laser sintering.

The earplug of Fig. 3 includes a customized hard shell 1000 with a faceplate 1001 at its outer (proximal) end. The faceplate includes an outer sound input opening 1032 provided with a mechanical peak clipper 1004, a multi-purpose cord adapter element 1014 with an in-situ measuring hole for optionally connecting the measuring hole to an external measuring tube 1024 or to a plug for closing the measuring hole in the normal operation of the earplug, and a sound inlet opening which is provided with a button 1002 which is manually operable in the direction 1003 to act as an attenuation button closing the sound inlet opening or as communication button opening the sound inlet opening for sound input into a sound passage 1036 which merges at its distal end with an in-situ measuring channel or tube 1016 which is acoustically connected to the measuring hole in adapter element 1014 and which extends to an inner sound opening 1034 at the inner end of the shell 1000. At the distal end of the resonance cavity 1008 a semi-integrated passive acoustic filter 1010 is provided. The tubes 1036 and 1016 are formed integral with the shell 1000. Further, also an insert cavity 1007 for a RFID (radio frequency identification device)-tag 1006 and an insert cavity 1012 for a detectable metal part 1013 are formed integral with the shell 1000. At the adapter element 1014 or at the plug for closing the measuring hole of the adapter element a cord fixation ring 1018 may be provided for fixing a neck cord 1020 at the shell 1000 for preventing loss of the earplug. The ring 1018 or the cord 1020 also may serve to manually pull the earplug in the axial direction 1022.

In the following these additional features and their functions will be explained in some detail.

The basic idea is to use rapid prototyping technology in a manufacturing environment as described, for example, in US 6,533,062 B1 or US 2003/0133583 A1. This technique is successfully being used to manufacture hearing aids and can be applied in a similar fashion for HPDs. By doing this, a whole new range of features and functions become feasible for HPDs.

The key to the following features and functions is this technology's capability to model and customize the earplug both to fit the individual shape of the ear, but also to utilize the given shape and volume for additional functionality. In some cases the processed earplug (using the mentioned rapid prototyping technology) becomes the chassis for the additional function (e.g. "RFID (radio frequency identification)", "HPD detection part", "multipurpose cord adapter") or the function is fully integrated (e.g. "intelligent HPD resonator"). The following list of functions and features indicates examples of application.

### Multipurpose cord adapter

In order to confirm acoustical performance of an HPD, an in-situ measurement tube is implemented to allow measurement of attenuation when the individual wears the device. Naturally this tube needs to be closed off during normal operation. The core element of this tube is the faceplate component referred to a multipurpose cord adapter 1014 that embodies several functions and features: fixation of external in-situ measurement probe tube 1024, one possible holder of the cord fixation ring 1018 for the neck cord 1020, holder of an ergonomic pull means (e.g. the cord fixation ring 1018,) for an inverse anatomy switch, holder of a plug for closing the in-situ tube during normal operation. If the element is made of metal it can serve as a metal component for detection purposes 1013 which in that case spares an extra insert cavity 1012. The design of the multipurpose cord adapter element 1014 is given extensive freedom (shape, material, insertion/removal concept, etc.) due to the base technology used for the faceplate portion of the earplug 1001.

### Semi-integrated passive filter

In passive HPDs acoustical filters mainly serve two purposes: firstly there is the defined amount of attenuation, secondly the filter can shape the frequency response of the attenuation in order to protect some frequencies while letting others through (e.g. block low frequency noise and let speech pass above 1 kHz). The proposed base technology enables both usages of predefined component placement geometries (e.g. cavities 1012 for metal component 1013 insertion) as well as semi-integration of functions where the material itself becomes part of the solution (e.g. insert cavities, acoustical filters). The semi-integrated passive filter 1010 is a structure of the second kind, where the tubes are made in shell material while the membranes are inserted components. Selection of membranes can be done to order and individual need, hence the component remains customizable. The filter must be considered and dimensioned together with other filter means like the customizable front chamber shaping structure (or resonance cavity) 1008, 1030 (Helmholtz resonator) and the mechanical peak clipper 1004.

### Communication/attenuation button

A core function of a passive HPD is to enable temporary audio bypass for purposes like listening to speech, alarm or other desired audio signals even though they are mixed with loud noise. This is often performed by a push/return-button opening a tube either bypassing the filter of the system or leading into the in-situ measurement probe tube 1016 on the inside of the closing plug to be connected to the adapter element 1014 when the measuring tube 1024 is removed. The integration of such a device into the faceplate 1001 overcomes many drawbacks of similar standard component solutions (e.g. complex tubing, acoustical leakage). An even more integrated solution is achieved by building the switch directly into the multipurpose cord adapter core element 1014 replacing the sealing plug. If the button is made of metal it could serve as a metal piece for the detection function, thereby eliminating the need for the separate metal part 1013.

### Inverse anatomy force button

A further level of integration of the on/off switch is based on the shell technology combined with the natural anatomy of the outer ear. In addition to a defined audio "leak" via a tube 1016 through the HPD, there is the alternative of creating a temporary leak between the device and the outer ear by slightly pulling the device out of the ear. This pull can be done by the cord 1020 or directly by grip and pull on the cord ring 1018. If the shell 1000 is shaped in an appropriate manner, the ear shape is such that the device will be naturally pulled back in place when the pull is relaxed.

### Mechanical peak clipping

Many applications for HPDs experience strong variations in noise exposure over time. The extreme example is people shooting with guns (military, hunters) where speech communication in-between the actions is strongly desired and where the sound gets very loud for a short time. In active devices such conditions have been solved with so-called "peak clippers" which are fairly easy to implement in electronics and which limit the output of the device independent of the input signal while leaving the signal undistorted for normal noise levels. For a passive device this can be realized by a pressure sensitive valve 1004 opening or blocking the audio canal at the sound inlet.

### Detectable HPD

HPDs are mostly used in industrial environments. In the food processing industry an additional requirement also affects these devices. Any foreign particle (to the food ingredients) must be detectable within the production process. For HPDs this implies that the devices need to contain a certain amount of metal to enable the detection equipment to find it if lost in the production line. Metal can be inserted into HPDs in a number of different ways. For example, metal can be mixed into the shell base material 1000, a specific metal component 1013 can be mounted in a prepared cavity 1012, the cord adapter faceplate element 1014 can be made of metal and the button part of the on/off switch 1002 can be made of metal. In a HPD with a RFID tag, the tag itself is detectable if the equipment for detection is implemented in the production line.

### HPD wearing compliance

Wearing of HPDs in industrial environments obliges to regulations in most countries. Assuming that the devices have the desired protective effect when they are worn (most other topics described address this very issue), the wearing itself becomes the compliance control topic. With recent developments in miniaturized RFID (radio frequency identification devices) technology, it becomes feasible to implement such devices into a customized HPD given the shell technology described. The RFID tag 1006 is inserted into a predefined cavity 1007 and when the wearer passes through gateways equipped with RFID detection systems, the positions of the two HPDs can be obtained and the control function carried out according to whether a predefined condition regarding the detected positions is fulfilled or not (e.g. separation of the HPDS according to the width of the head and height of the HPDs according to the ear height). As mentioned, the RFIDs can also serve as HPD detection devices in food production processes.

### Basic functions

Functions that conventionally are mounted components, such as a grip handle for insertion and removal of the HPD, can easily be integrated with use of the shell technology. The product design and assembly more and more becomes a software issue and the individual product is increasingly designed to order according to the specific requirements of each customer.

## Claims

1. A hearing protection earplug for being worn at least in part in the ear canal (28) of a person, comprising an integral shell (10, 1000) having an outer surface shaped for fitting at least in an outer part of the person's ear canal, an outer opening (18, 1032) for sound input, an acoustic filter element (20, 1010) within said shell for attenuating sound transmission through said earplug, and a resonance cavity (30, 1008) formed within said shell, said cavity being located between said outer opening and said filter element and being shaped as a resonator individually designed according to a desired sound frequency response of the person's outer ear and ear canal when wearing the earplug, **characterized by** said resonator comprising an inner mechanical structure (32, 34, 38, 1030) which is designed such that said resonator has a basic resonance frequency between 800 Hz and 5 kHz and such that the effective acoustic length (36) between said outer opening (18, 1032) and said filter element (20, 1010) is larger than the geometrical distance (37) between said outer opening and said filter element.

2. The earplug according to claim 1, wherein said inner mechanical structure (32, 34, 38, 1030) is designed such that the effective acoustic length (36) between said outer opening (18, 1032) and said filter element (20, 1010) is larger by at least 50% than the geometrical distance (37) between said outer opening and said filter element.

3. The earplug according to one of claims 1 or 2, wherein said inner mechanical structure (32, 34, 38, 1030) is designed such that the effective acoustic length (36) between said outer opening (18, 1032) and said filter element (20, 1010) corresponds to about the length of the person's ear canal (28).

4. The earplug according to one of the preceding claims, wherein said inner mechanical structure (32, 34, 1030) is a maze-like structure.

5. The earplug according to claim 4, wherein said inner mechanical structure comprises a plurality of axially spaced apart lamella-like elements (32, 1030) extending from an inner wall (12) of said shell (10, 1000) radially into said resonance cavity (30, 1008), wherein each lamella-like element has at least one sound opening (34) and wherein the sound openings of adjacent lamella-like elements are radially spaced apart from each other.

6. The earplug according to claim 5, wherein all of said sound openings (34) have about the same size.

7. The earplug according to claim 6, wherein all of said sound openings (34) have about the same size as said outer opening (18).

8. The earplug according to one of claims 1 to 3, wherein said inner mechanical structure is a curved tube (38).

9. The earplug according to one of the preceding claims, wherein said resonance cavity (30, 1008) is designed such that the sound frequency response of the person's outer ear and ear canal (28), when wearing the earplug, has a maximum between 800 Hz and 5 kHz.

10. The earplug according to claim 9, wherein said resonance cavity (30, 1008) is designed such that the sound frequency response of the person's outer ear and ear canal (28), when wearing the earplug, is substantially equal to the corresponding natural sound frequency response of the person's outer ear and ear canal when not wearing the earplug.

11. The earplug according to one of the preceding claims, wherein the distal end of said resonance cavity (30) is formed by a wall (14) having an opening in which said filter element (20) is located.

12. The earplug according to one of the preceding claims, wherein said shell (10, 1000) comprises a second cavity (22) located between said filter element (20, 1010) and an inner opening (24), said inner opening being designed for facing the person's eardrum (26).

13. The earplug according to claim 12, wherein said filter element (20, 1010) is designed for acoustically connecting said resonance cavity (30, 1008) and said second cavity (22).

14. The earplug according to one of the preceding claims, wherein said filter element (20, 1010) is a tube or diaphragm filter.

15. The earplug according to one of the preceding claims, wherein said outer surface of said shell (10, 1000) is individually shaped according to the measured inner shape of the person's outer ear and ear canal (28).

16. The earplug according to claim 15, wherein said shell (10, 1000) has an elasticity of from shore D85 to shore D65.

17. The earplug according to one of claims 15 or 16, wherein said shell (20, 1000) is made of polyamide.

18. The earplug according to one of the preceding claims, wherein said outer sound input opening (18, 1032) is located at the outer end (1001) of said shell (10, 1000) and said resonance cavity (30, 1008) does not extend outwardly beyond said outer sound input opening.

19. A method for manufacturing a hearing protection earplug for being worn at least in part in the ear canal (28) of a person, comprising: measuring the inner shape of the person's outer ear and ear canal (28); determining a desired sound frequency response function of the person's outer ear and ear canal when wearing the earplug; and forming a shell (10, 1000) having an acoustic filter element (20, 1010) within said shell for attenuating sound transmission through said earplug and a resonance cavity (30, 1008) individually shaped, by using said measured inner shape of the person's outer ear and ear canal, in order to tune the sound frequency response of the earplug according to said desired sound frequency response of the person's outer ear and ear canal when wearing the earplug, wherein said shell (10, 1000) is formed with an outer opening (18, 1032) for sound input, wherein said resonance cavity (30, 1008) is formed between said outer opening and said filter element (20, 1010) and is shaped as a resonator, said resonator comprising an inner mechanical structure (32, 34, 38, 1030) which is designed such that said resonator has a basic resonance frequency between 800 Hz and 5 kHz and such that the effective acoustic length (36) between said outer opening (18, 1032) and said filter element (20, 1010) is larger than the geometrical distance (37) between said outer opening and said filter element.

20. The method according to claim 19, wherein the outer surface of said shell (10, 1000) is individually shaped according to said measured inner shape of the person's outer ear and ear canal (28) for optimized fit.

21. The method of one of claims 19 or 20, wherein said shell (10, 1000) is built-up by an additive process.

22. The method of claim 21, wherein said shell (10, 1000) is formed by layer-by-layer laser sintering of a powder material.

23. The method of one of claims 19 to 22, further comprising establishing an acoustic model of the person's ear when wearing the earplug; and determining by said model a set of geometrical parameters of the earplug from said measured shape of the person's outer ear and ear canal (28) and said desired sound frequency response; wherein said resonance cavity (30, 1008) is individually shaped according to at least some of said geometrical parameters.

24. The method according to claim 23, wherein the outer surface of said shell (10, 1000) is individually shaped according to at least some of said geometrical parameters for optimized fit.

25. The method according to one of claims 23 or 24, further comprising: determining a hearing loss function of the person's ear as further input to said acoustic model for determining said geometric parameters.

26. The method according to one of claims 23 to 25, wherein said geometric parameters include the cross section of said outer opening (18, 1032), the volume of said resonance cavity (30, 1008) and the effective acoustic length (36) between said outer opening and said filter element (20, 1010).

## Patentansprüche

1. Gehörschutzohrstopfen, der mindestens zum Teil im Gehörgang (28) einer Person zu tragen ist, mit einer einstückigen Schale (10, 1000) mit einer Außenfläche, die so geformt ist, dass sie mindestens zum Teil in einen äußeren Teil des Gehörgangs der Person passt, einer äußeren Schalleinlassöffnung (18, 1032), einem akustischen Filterelement (20, 1010) innerhalb der Schale zum Abschwächen der Schallübertragung durch den Ohrstopfen hindurch, sowie einem innerhalb der Schale ausgebildeten Resonanzhohlraum (30, 1008), der zwischen der äußeren Öffnung und dem Filterelement angeordnet ist und als ein Resonator geformt ist, der individuell gemäß einem gewünschten Schallfrequenzgang des Außenohrs und des Gehörgangs der Person beim Tragen des Ohrstopfens ausgelegt ist, **dadurch gekennzeichnet, dass** der Resonator eine innere mechanische Struktur (32, 34, 38, 1030) aufweist, die so ausgelegt ist, dass der Resonator eine Grundresonanzfrequenz zwischen 800 Hz und 5 kHz aufweist und die effektive akustische Länge (36) zwischen der äußeren Öffnung (18, 1032) und dem Filterelement (20, 1010) größer als die geometrische Distanz (37) zwischen der äußeren Öffnung und dem Filterelement ist.

2. Ohrstopfen gemäß Anspruch 1, wobei die innere mechanische Struktur (32, 34, 38, 1030) so ausgelegt ist, dass die effektive akustische Länge (36) zwischen der äußeren Öffnung (18, 1033) und dem Filterelement (20, 1010) wenigstens 50% größer als die geometrische Distanz (37) zwischen der äußeren Öffnung und dem Filterelement ist.

3. Ohrstopfen gemäß Anspruch 1 oder 2, wobei die innere mechanische Struktur (32, 34, 38, 1030) so ausgelegt ist, dass die effektive akustische Länge (36) zwischen der äußeren Öffnung (18, 1032) und dem Filterelement (20, 1010) in etwa der Länge des Gehörgangs (28) der Person entspricht.

4. Ohrstopfen gemäß einem der vorhergehenden Ansprüche, wobei die innere mechanische Struktur (32, 34, 1030) eine labyrinthartige Struktur ist.

5. Ohrstopfen gemäß Anspruch 4, wobei die innere mechanische Struktur eine Mehrzahl von axial beabstandeten lamellenartigen Elementen (32, 1030) aufweist, die sich von einer Innenwand (12) der Schale (10, 1000) aus in radialer Richtung in den Resonanzhohlraum (30, 1008) erstrecken, wobei jedes lamellenartige Element mindestens eine Schallöffnung (34) aufweist und wobei die Schallöffnungen benachbarter lamellenartiger Elemente in radialer Richtung voneinander beabstandet sind.

6. Ohrstopfen gemäß Anspruch 5, wobei alle Schallöffnungen (34) in etwa die gleiche Größe haben.

7. Ohrstopfen gemäß Anspruch 6, wobei alle Schallöffnungen (34) in etwa die gleiche Größe wie die äußere Öffnung (18) haben.

8. Ohrstopfen gemäß einem der Ansprüche 1 bis 3, wobei es sich bei der inneren mechanischen Struktur um eine gekrümmte Röhre (38) handelt.

9. Ohrstopfen gemäß einem der vorhergehenden Ansprüche, wobei der Resonanzhohlraum (30, 1008) so ausgelegt ist, dass der Schallfrequenzgang des Außenohrs und des Gehörgangs (28) der Person beim Tragen des Ohrstopfens ein Maximum zwischen 800 Hz und 5 kHz hat.

10. Ohrstopfen gemäß Anspruch 9, wobei der Resonanzhohlraum (30, 1008) so ausgelegt ist, dass der Schallfrequenzgang des Außenohrs und des Gehörgangs (28) der Person beim Tragen des Ohrstopfens im wesentlichen gleich dem entsprechenden natürlichen Schallfrequenzgang des Außenohrs und des Gehörgangs der Person beim Nicht-Tragen des Ohrstopfens ist.

11. Ohrstopfen gemäß einem der vorhergehenden Ansprüche, wobei das distale Ende des Resonanzhohlraums (30) durch eine Wand (14) mit einer Öffnung, in welcher das Filterelement (20) angeordnet ist, gebildet wird.

12. Ohrstopfen gemäß einem der vorhergehenden Ansprüche, wobei die Schale (10, 1000) einen zweiten Hohlraum (22) aufweist, der zwischen dem Filterelement (20, 1010) und einer inneren Öffnung angeordnet ist, die so ausgelegt ist, dass sie dem Trommelfell (26) der Person gegenüber liegt.

13. Ohrstopfen gemäß Anspruch 12, wobei das Filterelement (20, 1010) ausgelegt ist, um den Resonanzhohlraum (30, 1008) und den zweiten Hohlraum (22) akustisch zu verbinden.

14. Ohrstopfen gemäß einem der vorhergehenden Ansprüche, wobei es sich bei dem Filterelement (20, 1010) um einen Rohr- oder Membranfilter handelt.

15. Ohrstopfen gemäß einem der vorhergehenden Ansprüche, wobei die Außenfläche der Schale (10, 1000) gemäß der gemessenen Innenform des Außenohrs und des Gehörgangs (28) der Person individuell geformt ist.

16. Ohrstopfen gemäß Anspruch 15, wobei die Schale (10, 1000) eine Elastizität von Shore D85 bis Shore D65 aufweist.

17. Ohrstopfen gemäß einem der Ansprüche 15 oder 16, wobei die Schale (20) aus Polyamid gefertigt ist.

18. Ohrstopfen gemäß einem der vorhergehenden Ansprüche, wobei die äußerer Schalleinlassöffnung (18, 1032) an dem äußeren Ende (1001) der Schale (10, 1000) angeordnet ist und sich der Resonanzhohlraum (30, 1008) nach außen nicht über die äußere Schalleinlassöffnung hinaus erstreckt.

19. Verfahren zum Herstellen eines Gehörschutzohrstopfens, der zumindest zum Teil im Gehörgang (28) einer Person zu tragen ist, wobei im Zuge des Verfahrens: die Innenform des Außenohrs und des Gehörgangs (28) der Person gemessen wird; ein gewünschter Schallfrequenzgang des Außenohrs und des Gehörgangs der Person beim Tragen des Ohrstopfens festgelegt wird; und eine Schale (10, 1000) mit einem akustischen Filterelement (20, 1010) innerhalb der Schale zum Abschwächen der Schallübertragung durch den Ohrstopfen hindurch und einem individuell geformten Resonanzhohlraum (30, 1008) hergestellt wird, indem die gemessene Innenform des Außenohrs und des Gehörgangs der Person verwendet wird, um den Schallfrequenzgang des Ohrstopfens gemäß dem gewünschten Schallfrequenzgang des Außenohrs und des Gehörgangs der Person beim Tragen des Ohrstopfens einzustellen, wobei die Schale (10, 1000) mit einer äußeren Schalleinlassöffnung (18, 1032) gebildet wird, wobei der Resonanzhohlraum (30, 1008) zwischen der äußeren Öffnung und dem Filterelement (20, 1010) gebildet wird und als Resonator geformt wird, der eine innere mechanische Struktur (32, 34, 38, 1030) aufweist, die so ausgelegt ist, dass der Resonator eine Grundresonanzfrequenz zwischen 800 Hz und 5 kHz aufweist und die effektive akustische Länge (36) zwischen der äußeren Öffnung (18, 1032) und dem Filterelement (20, 1010) größer als die geometrische Distanz (37) zwischen der äußeren Öffnung und dem Filterelement ist.

20. Verfahren gemäß Anspruch 19, wobei die Außenfläche der Schale (10, 1000) gemäß der gemessenen Innenform des Außenohrs und des Gehörgangs (28) der Person individuell für optimierte Passung geformt wird.

21. Verfahren gemäß einem der Ansprüche 19 oder 20, wobei die Schale (10, 1000) mittels eines additiven Prozesses aufgebaut wird.

22. Verfahren gemäß Anspruch 21, wobei die Schale (10, 1000) mittels schichtweisem Lasersintern eine Pulvermaterials gebildet wird.

23. Verfahren gemäß einem der Ansprüche 19 bis 22, wobei ferner ein akustisches Modell des Ohrs der Person beim Tragen des Ohrstopfens erzeugt wird und mittels des Modells ein Satz geometrischer Parameter des Ohrstopfens aus der gemessenen Form des Außenohrs und des Gehörgangs (28) der Person und dem gewünschten Schallfrequenzgang bestimmt wird, wobei der Resonanzhohlraum (30, 1008) gemäß mindestens einigen der geometrischen Parameter individuell geformt wird.

24. Verfahren gemäß Anspruch 23, wobei die Außenfläche der Schale (10, 1000) gemäß mindestens einigen der geometrischen Parameter zwecks optimierter Passung individuell geformt wird.

25. Verfahren gemäß einem der Ansprüche 23 oder 24, wobei ferner eine Gehörverlustfunktion des Gehörs der Person als weiterer Input für das akustische Modell zum Bestimmen der geometrischen Parameter bestimmt wird.

26. Verfahren gemäß einem der Ansprüche 23 bis 25, wob ei die geometrischen Parameter den Querschnitt der äußeren Öffnung (18, 1032), das Volumen des Resonanzhohlraums (30, 1008) und die effektive akustische Länge (36) zwischen der äußeren Öffnung und dem Filterelement (20,1010) umfassen.

## Revendications

1. Bouchon auriculaire pour la protection de l'ouïe destiné à être porté au moins en partie dans le canal auditif (28) d'une personne, comportant une coque intégrale (10, 1000) ayant une surface extérieure configurée pour s'ajuster dans au moins une partie extérieure du canal auditif d'une personne, une ouverture extérieure (18, 1032) pour l'entrée des sons, un élément à filtre acoustique (20, 1010) à l'intérieur de ladite coque pour atténuer la transmission des sons à travers ledit bouchon auriculaire, et une cavité à résonance (30, 1008) formée à l'intérieur de ladite coque, ladite cavité étant placée entre ladite ouverture extérieure et ledit élément à filtre et étant configuré en un résonateur conçu individuellement en fonction d'une réponse souhaitée à la fréquence des sons de l'oreille externe et du canal auditif d'une personne lorsqu'elle porte le bouchon auriculaire, **caractérisé en ce que** ledit résonateur comporte une structure mécanique intérieure (32, 34, 38, 1030) qui est conçue de façon que ledit résonateur ait une fréquence de résonance de base comprise entre 800 Hz et 5 kHz et que la longueur acoustique effective (36) entre ladite ouverture extérieure (18, 1032) et ledit élément à filtre (20, 1010) soit plus grande que la distance géométrique (37) entre ladite ouverture extérieure et ledit élément à filtre.

2. Bouchon auriculaire selon la revendication 1, dans lequel ladite structure mécanique intérieure (32, 34, 38, 1030) est conçue de façon que la longueur acoustique effective (36) entre ladite ouverture extérieure (18, 1032) et ledit élément à filtre (20, 1010) soit supérieure d'au moins 50 % à la distance géométrique (37) entre ladite ouverture extérieure et ledit élément à filtre.

3. Bouchon auriculaire selon l'une des revendications 1 et 2, dans lequel ladite structure mécanique intérieure (32, 34, 38, 1030) est conçue de façon que la longueur acoustique effective (36) entre ladite ouverture extérieure (18, 1032) et ledit élément à filtre (20, 1010) corresponde à environ la longueur du canal auditif (28) de la personne.

4. Bouchon auriculaire selon l'une des revendications précédentes, dans lequel ladite structure mécanique (32, 34, 1030) est une structure analogue à un labyrinthe.

5. Bouchon auriculaire selon la revendication 4, dans lequel ladite structure mécanique intérieure comporte de multiples éléments (32, 1030) analogues à des lamelles espacés axialement s'étendant depuis une paroi intérieure (12) de ladite coque (10, 1000) radialement dans ladite cavité à résonance (30, 1008), dans lequel chaque élément analogue à une lamelle présente au moins une ouverture (34) pour les sons et dans lequel les ouvertures pour les sons d'éléments adjacents analogues à des lamelles sont espacées radialement les unes des autres.

6. Bouchon auriculaire selon la revendication 5, dans lequel toutes lesdites ouvertures (34) pour les sons ont approximativement la même dimension.

7. Bouchon auriculaire selon la revendication 6, dans lequel toutes lesdites ouvertures (34) pour les sons ont approximativement la même dimension que ladite ouverture extérieure (18).

8. Bouchon auriculaire selon l'une des revendications 1 à 3, dans lequel ladite structure mécanique intérieure est un tube courbé (38).

9. Bouchon auriculaire selon l'une des revendications précédentes, dans lequel ladite cavité à résonance (30, 1008) est conçue de façon que la sensibilité à la fréquence des sons de l'oreille externe et du canal auditif (28) de la personne, lorsqu'elle porte le bouchon auriculaire ait un maximum compris entre 800 Hz et 5 kHz.

10. Bouchon auriculaire selon la revendication 9, dans lequel ladite cavité à résonance (30, 1008) est conçue de façon que la sensibilité à la fréquence des sons de l'oreille externe et du canal auditif (28) de la personne, lorsqu'elle porte le bouchon auriculaire, soit sensiblement égale à la sensibilité naturelle correspondante à la fréquence des sons de l'oreille externe et du canal auditif de la personne lorsqu'elle ne porte pas le bouchon auriculaire.

11. Bouchon auriculaire selon l'une des revendications précédente, dans lequel l'extrémité distale de ladite cavité à résonance (30) est formée par une paroi (14) ayant une ouverture dans laquelle est placé ledit élément à filtre (20).

12. Bouchon auriculaire selon l'une des revendications précédentes, dans lequel ladite coque (10, 1000) comporte une seconde cavité (22) placée entre ledit élément à filtre (20, 1010) et une ouverture intérieure (24), ladite ouverture intérieure étant conçue pour faire face au tympan (26) de la personne.

13. Bouchon auriculaire selon la revendication 12, dans lequel ledit élément à filtre (20, 1010) est conçu pour relier acoustiquement ladite cavité à résonance (30, 1008) et ladite seconde cavité (22).

14. Bouchon auriculaire selon l'une des revendications précédentes, dans lequel ledit élément à filtre (20, 1010) est un filtre à tube ou à membrane.

15. Bouchon auriculaire selon l'une des revendications précédentes, dans lequel ladite surface extérieure de ladite coque (10, 1000) est configurée individuellement conformément à la forme intérieure mesurée de l'oreille externe et du canal auditif (28) de la personne.

16. Bouchon auriculaire selon la revendication 15, dans lequel ladite coque (10, 1000) a une élasticité allant de Shore D85 à Shore D65.

17. Bouchon auriculaire selon l'une des revendications 15 et 16, dans lequel ladite coque (20, 1000) est réalisée en polyamide.

18. Bouchon auriculaire selon l'une des revendications précédentes, dans lequel ladite ouverture extérieure (18, 1032) d'entrée des sons est située à l'extrémité extérieure (1001) de ladite coque (10, 1000) et ladite cavité à résonance (30, 1008) ne s'étend pas vers l'extérieur au-delà de ladite ouverture extérieure d'entrée des sons.

19. Procédé de fabrication d'un bouchon auriculaire pour la protection de l'ouïe destiné à être porté dans au moins une partie du canal auditif (28) d'une personne, comprenant : la mesure de la forme intérieure de l'oreille externe et du canal auditif (28) de la personne ; la détermination d'une fonction de sensibilité souhaitée à la fréquence des sons de l'oreille externe et du canal auditif de la personne lorsqu'elle porte le bouchon auriculaire ; et la formation d'une coque (10, 1000) ayant un élément à filtre acoustique (20, 1010) à l'intérieur de ladite coque pour atténuer la transmission des sons à travers ledit bouchon auriculaire et une cavité à résonance (30, 1008) configurée individuellement, par l'utilisation de ladite forme intérieure mesurée de l'oreille externe et du canal auditif de la personne, afin d'accorder la sensibilité à la fréquence des sons du bouchon auriculaire conformément à ladite sensibilité souhaitée à la fréquence des sons de l'oreille externe et du canal auditif de la personne lorsqu'elle porte le bouchon auriculaire, dans lequel ladite coque (10, 1000) est formée de façon à avoir une ouverture extérieure (18, 1032) pour l'entrée des sons, dans lequel ladite cavité à résonance (30, 1008) est formée entre ladite ouverture extérieure et ledit élément à filtre (20, 1010) et est configurée en un résonateur, ledit résonateur comportant une structure mécanique intérieure (32, 34, 38, 1030) qui est conçue de façon que ledit résonateur ait une fréquence de résonance de base comprise entre 800 Hz et 5 kHz et que la longueur acoustique effective (36) entre ladite ouverture extérieure (18, 1032) et ledit élément à filtre (20, 1010) soit supérieure à la distance géométrique (37) entre ladite ouverture extérieure et ledit élément à filtre.

20. Procédé selon la revendication 19, dans lequel la surface extérieure de ladite coque (10, 1000) est configurée individuellement en fonction de ladite forme intérieure mesurée de l'oreille externe et du canal auditif (28) de la personne pour un ajustement optimisé.

21. Procédé selon l'une des revendications 19 et 20, dans lequel ladite coque (10, 1000) est construite par un processus additif.

22. Procédé selon la revendication 21, dans lequel ladite coque (10, 1000) est formée par un frittage couche par couche au laser d'une matière en poudre.

23. Procédé selon l'une des revendications 19 à 22, comprenant en outre l'établissement d'un modèle acoustique de l'oreille de la personne lorsqu'elle porte le bouchon auriculaire ; et la détermination par ledit modèle d'un ensemble de paramètres géométriques du bouchon auriculaire à partir de ladite forme mesurée de l'oreille externe et du canal auditif (28) de la personne et ladite sensibilité souhaitée à la fréquence des sons ; dans lequel ladite cavité à résonance (30, 1008) est configurée individuellement en fonction d'au moins certains desdits paramètres géométriques.

24. Procédé selon la revendication 23, dans lequel la surface extérieure de ladite coque (10, 1000) est configurée individuellement en fonction d'au moins certains desdits paramètres géométriques pour un ajustement optimisé.

25. Procédé selon l'une des revendications 23 et 24, comprenant en outre : la détermination d'une fonction de perte d'audition de l'oreille de la personne en tant qu'autre entrée pour ledit modèle acoustique pour la détermination desdits paramètres géométriques.

26. Procédé selon l'une des revendications 23 à 25, dans lequel lesdits paramètres géométriques comprennent la section transversale de ladite ouverture extérieure (18, 1032), le volume de ladite cavité à résonance (30, 1008) et la longueur acoustique effective (36) entre ladite ouverture extérieure et ledit élément à filtre (20, 1010).
